# EUROPEAN PATENT APPLICATION

(11) **EP 3 783 619 A1**
(43) Date of publication of application: **24.02.2021**
(21) Application number: 18926215.7
(22) Date of filing: 17.08.2018
(51) Int. Cl.: G16H 50/30, G16H 50/70

(54) **HUMAN BODY HEALTH ASSESSMENT METHOD AND SYSTEM BASED ON SLEEP BIG DATA**

(30) Priority: 13.07.2018 CN 201810772360
(71) Applicant: Zhejiang Yangtze Delta Region Institute of Tsinghua University, Jiaxing, Zhejiang 314016 (CN); Keeson Technology Corporation Limited, Jiaxing, Zhejiang 314016 (CN)
(72) Inventor: YANG, Xiangdong, Jiaxing, Zhejiang 314016 (CN); SHAN, Huafeng, Jiaxing, Zhejiang 314016 (CN); CAO, Hui, Jiaxing, Zhejiang 314016 (CN); SONG, Qilong, Jiaxing, Zhejiang 314016 (CN); LI, Hongwen, Jiaxing, Zhejiang 314016 (CN); ZHU, Zhenyu, Jiaxing, Zhejiang 314016 (CN); HAN, Xiuping, Jiaxing, Zhejiang 314016 (CN)
(74) Representative: Viering, Jentschura & Partner mbB Patent- und Rechtsanwälte
(86) International application number: PCT/CN2018/100955
(87) International publication number: WO 2020/010668

(57) **Abstract**

Disclosed is a human body health assessment method and assessment system based on sleep big data. The method and the system acquire biometric data of a human body in a long-term, stable, and ever-increasing manner, and clearly and definitely describe the physical conditions of a human being and predict future health thereof using advanced data mining technology. The method comprises: acquiring, by a sensor mounted on a bed, various physiological data of a human body during sleep and storing the same to a cloud server (101); and obtaining a physiological evaluation index using an artificial intelligence learning model and generating a health status analysis report of the human body. By acquiring the data of the sensor mounted on the bed, data training of the artificial intelligence learning model is performed, to allow the model to automatically learn features related to the health of the human body, thereby reducing time costs for manual selection and construction of features, and achieving the purpose of accurate prediction of diseases. By further training a classifier model, a disease type can be directly acquired from a health assessment report.

## Description

### Field of the Invention

The present disclosure relates to the technical field of sleep health data analysis and management, and in particular to a human health assessment method and assessment system based on sleep big data.

### Background of the Invention

Commonly used physical health assessment methods at present mainly include performing questionnaire surveys, testing various indexes of the body by means of medical devices, wearing wearable devices with physiological data, etc. Among these methods, in a questionnaire survey, users participating in the survey answer, based on their physical status in daily life, a health assessment questionnaire provided by the medical staff, who evaluates the health status of the users after collecting the questionnaire filled in thereby. To detect physiological data by medical devices or wearable devices, it is necessary to record the test results regularly and find out health problems timely according to the recorded test data. The authenticity of the data source used by these assessment methods is unreliable. As the data acquisition process is easily affected by user awareness, it is difficult to completely record the physiological data in normal life and habits and to ensure the standard and accuracy of data collection.

Sleep accounts for 1/3 of people's daily life, and the various physiological data of a human body in a sleep state are relatively stable. Collecting human physiological data in the sleep state for health assessment can ensure the accuracy, stability, and persistence of data and provide accuracy of an evaluation result. Currently, the method medically recognized for judging the sleep state in clinical applications is the sleep clustering method. In the sleep clustering method, brain waves, eye movements, and myoelectricity are measured, and the sleep state can be judged by these waves. However, according to the method of measuring brain waves, myoelectricity, and the like, it is necessary to mount an electrode to a testee, which is a heavy load on a tester. Therefore, it is impossible to measure brain waves, myoelectricity, or the like in an ordinary family.

Physical assessment is an organized and systematic collection process of objective data. Based on health history and head to toe or general system examination, good physical assessment results need an organized and systematic method to explore and study direct reactions and actual or potential problems of users. Therefore, how to ensure a sufficient amount and sufficiently comprehensive physiological data collection from the users in an early stage, and how to ensure the rationality of data processing to reduce errors are issues requiring great concerns in human health assessment.

### Summary of the Invention

The purpose of the present disclosure is to provide a human health assessment method and assessment system based on sleep big data in view of the shortcomings in the prior art. The health big data research in the system refers to a long-term, stable, and continuously growing acquisition of human life characteristic data, clear and definite description of the physical state of human individuals by advanced data mining technology, and prediction of future health thereof. By acquiring data from a sensor mounted on a bed, and performing data training on an artificial intelligence learning model to allow the model to automatically learn features related to human health, the time costs of manual selection and construction of features can be saved, thus achieving the purpose of accurate prediction of diseases. Further, by training a classifier model, a disease type can be directly obtained in a health assessment report.

In order to achieve the above purpose, the present disclosure adopts the technical solution of a human health assessment method based on sleep big data, comprising the steps of:
acquiring various physiological data of a human body during sleep by a sensor mounted on a bed, and storing these sleep data permanently in a cloud server;
preprocessing the sleep data stored in the cloud server to filter out missing and incorrect data, and inputting correct data into an artificial intelligence learning model for training, such that the artificial intelligence learning model learns disease features and calculates to obtain a physiological evaluation index;
training a classifier model using the disease features of data learnt by the above artificial intelligence learning model, such that the classifier model can identify disease types corresponding to different data; and
generating a human health status analysis report according to the human physiological evaluation index and the classifier model obtained in the above data training.

Further, in some embodiments, the correct data after preprocessing in the above method is divided into sleep data with a disease tag and sleep data without a disease tag, the sleep data with a disease tag coming from a human body suffering from a known disease type while the sleep data without a disease tag coming from a human body whose health condition is not known.

Further, in an embodiment, the above process of inputting the correct data into the artificial intelligence learning model for training comprises:
sending the sleep data without a disease tag after preprocessing to an autoencoder network to calculate and obtain an unsupervised network loss;
sending the sleep data with a disease tag after preprocessing to an autoencoder network having the same parameters as the above autoencoder network, to obtain intermediate layer dimension reduction output data and calculate to obtain a supervised network loss; and
iteratively training above two steps, and stopping iteration until a sum of the unsupervised network loss and the supervised network loss as obtained no longer changes or reaches maximum iterations, sleep data obtained then being used as the physiological evaluation index.

Further, in an embodiment, the intermediate layer dimension reduction output data obtained from the sleep data with a disease tag in the autoencoder network is input into a classifier to train the classifier model.

Further, the physiological evaluation index is used to evaluate whether the human body is in a sick state, and the classifier model is used to analyze a disease type of the human body.

In order to achieve the above purpose, a further technical solution adopted by the present disclosure is a human health assessment system based on sleep big data, the system comprises:
a sleep data acquisition unit for acquiring various physiological data of a human body during sleep, the data coming from a sensor mounted on a bed;
a sleep data storage unit, including a cloud server for storing all sleep data collected by the sleep data acquisition unit;
a data training unit for preprocessing sleep data and training data by an artificial intelligence learning model to acquire a physiological evaluation index;
a classifier model training unit for performing classifier model training on the sleep data trained by the artificial intelligence learning model; and
a report generation unit for generating a human health status analysis report according to the human physiological evaluation index and the classifier model as obtained based on data training.

Further, in a specific embodiment, the data training unit comprises:
a data preprocessor for filtering out missing and incorrect sleep data stored in the cloud server; and
an autoencoder for creating an autoencoder network, and iteratively training data by calculating a network loss.

In order to achieve the above purpose, a further technical solution adopted by the present disclosure comprises a terminal device, the device comprising the above human health assessment system based on sleep big data. The device further comprises an operating mechanism for controlling the above report generation unit to generate a report.

The advantages of the present disclosure are as follows:
1. The system and the sensor mounted on the bed effectuate data transmission. The service life of the bed is long, and the physiological data of the user in the sleep state are relatively stable, thus ensuring persistence and stability of data acquisition. On the other hand, the massive sleep data obtained by the sensor mounted on the bed provides a high-quality data basis for health assessment.
2. The cloud server of the system of the present disclosure is used to store a user's daily sleep data. This ensures the stability and security of the sleep data and meanwhile provides a large amount of data basis for health assessment, improving the accuracy of evaluation results.
3. The method of the present disclosure performs artificial intelligence learning model training by acquiring a large number of physiological data of a human body during sleep. The active consciousness of a human being is very weak in a sleep state, such that the physiological data obtained at this moment can truly reflect the health state of the human body. By building an applicable artificial intelligence learning model, the model can automatically learn the features related to human health, thereby saving time costs in artificial selection and construction of features and achieving the purpose of accurate prediction of diseases.

### Brief Description of the Drawings

In order to better understand the purpose, features, and advantages of the present disclosure, preferred embodiments of the present disclosure will be described in detail in conjunction with the accompanying drawings. Obviously, the drawings described below are only some embodiments of the present disclosure. For a person of ordinary skill in the art, other drawings can be obtained according to these drawings without incurring any creative labor. In the drawings:
Fig. 1 schematically shows a flow chart of a human health assessment method based on sleep big data in an embodiment of the present disclosure;
Fig. 2 schematically shows a flow chart of performance of data training by an artificial intelligence learning model in an embodiment of the present disclosure;
Fig. 3 schematically shows a flow chart of training of a classifier model in an embodiment of the present disclosure; and
Fig. 4 shows a frame diagram of a human health assessment system based on sleep big data in an embodiment of the present disclosure.

### Detailed Description of the Embodiments

In order to enable a person in the art to better understand the technical solutions of the present disclosure, the technical solutions in the embodiments of the present disclosure will be clearly and fully described in conjunction with the drawings in the embodiments of the present disclosure. Obviously, the embodiments described are only portions, rather than all of the embodiments of the present disclosure. Based on the embodiments in the present disclosure, all other embodiments obtained by a person of ordinary skill in the art without incurring any creative labor shall fall within the protection scope of the present disclosure.

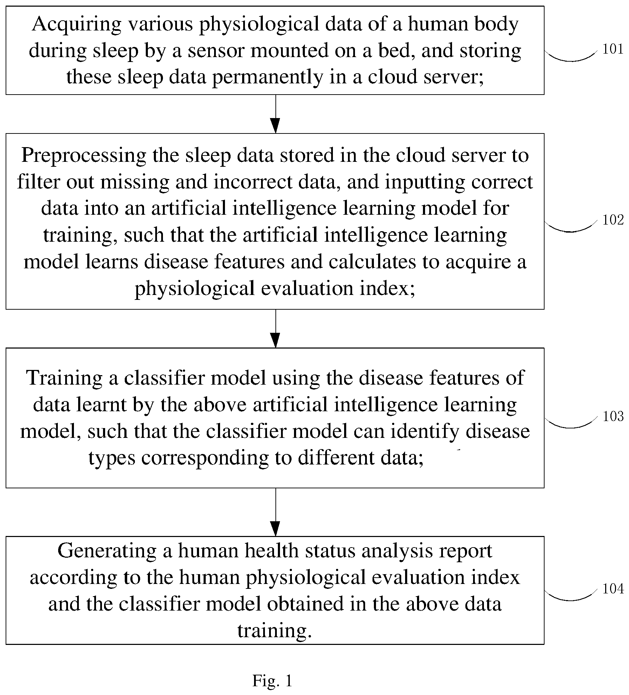

In an embodiment of the present disclosure, a human health assessment method based on sleep big data is provided, for improving the accuracy of health assessment results and achieving the purpose of accurate prediction of diseases. Beds suitable for mounting sensors include but are not limited to: electric beds, intelligent beds, beds with induction mattresses, etc. Fig. 1 shows a flow chart of a human health assessment method based on sleep big data in an embodiment of the present disclosure. As shown in Fig. 1, in the embodiment of the present disclosure, the human health assessment method based on sleep big data can comprises the following steps.

In step 101, various physiological data of a human body during sleep are obtained by a sensor mounted on a bed, and these sleep data are permanently stored in a cloud server.

As can be seen from the flow chart shown in Fig. 1, the human health assessment method based on sleep big data in the embodiment of the present disclosure processes, analyzes, and trains massive sleep data stored in the cloud server using a data training process by means of an artificial intelligence learning model. In this method, data transmission is realized by the cloud server and the sensor mounted on the bed. For an electric bed or intelligent bed containing an electric control system, the cloud server can also communicate with a database of the control system to acquire sleep data of users in real time and stably, which provides a high-quality data basis for health assessment. The types of the various physiological data stored in the cloud server during sleep are associated with the sensor types mounted on the bed. In practical applications, specific sensors can be selected to acquire target data according to the focus of health assessment. For example, the physiological data obtained in the present embodiment include: heart rate, respiratory rate, turning over, fretting, snoring, and leaving bed information.

In step 102, the sleep data stored in the cloud server is preprocessed to filter out missing and incorrect data, and to put correct data into the artificial intelligence learning model for training, such that the artificial intelligence learning model can learn the disease features and calculate to obtain a physiological evaluation index.

The original human sleep data stored in the cloud server is acquired in real time without any processing, and would inevitably contain a large number of missing and incorrect data. Therefore, before data analysis, data preprocessing must be performed to screen out correct sleep data, which can then be put into the artificial intelligence learning model for training. Otherwise, the whole model will be disordered, thereby preventing any correct prediction results from being obtained. While filtering out noise data during data preprocessing, sleep data is divided into sleep data with a disease tag and sleep data without a disease tag according to the data source, wherein the sleep data with a disease tag refers to physiological data collected from a human body suffering from known disease types, while the sleep data without a disease tag is physiological data collected from a human body whose health condition is not known. Preprocessed sleep data will be sent to the artificial intelligence learning model for training, and can then be used to assess the health status of the human body. In specific implementation, when the sleep data a with disease tag is collected for artificial intelligence learning model training and classifier model training, an individual who is in a disease state with an accurately known disease type will be selected. This individual's sleep data will be collected and input into the artificial intelligence learning model, for the model to identify whether the individual is in a normal or sick state and to identify the disease type if in a sick state. When the sleep data without a disease tag is collected for artificial intelligence learning model training, an individual whose health condition is not known will be selected. The sleep data of this individual will be collected, input into the artificial intelligence learning model, and summarized, such that the model learns the commonality of human sleep data.

In step 103, a classifier model is trained using the disease features of data learnt by the above artificial intelligence learning model, such that the classifier model can identify disease types corresponding to different data.

In the present application, the data trained by the artificial intelligence learning model will be used as the physiological evaluation index, and can be used to identify whether an individual is in a sick state or a normal state. Furthermore, the sleep data with a disease tag, after being output at reduced dimension in an intermediate layer of an autoencoder network, is used as an input of an SVM classifier to train the classifier model, such that the classifier model can identify disease types corresponding to different data. Through step 102, the artificial intelligence learning model can learn some features related to the disease from the original data, and then through step 103, the classifier model is further trained to be able to identify the specific type of disease of the individual generating the sleep data.

In step 104, a human health status analysis report is generated according to the human physiological evaluation index and the classifier model obtained from the above data training.

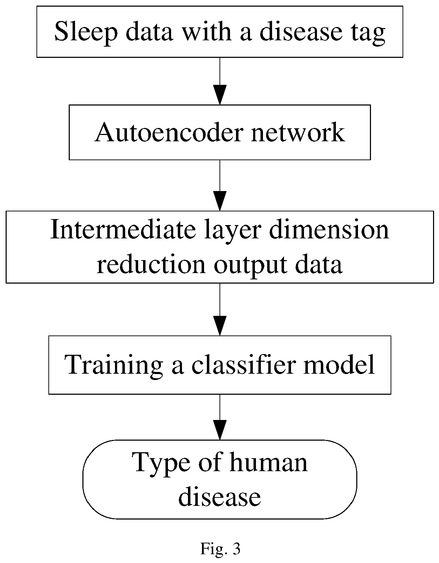

A specific embodiment of the present disclosure will be described below in conjunction with Figs. 1-3.

First, sleep data stored in a cloud server is preprocessed to filter out missing and incorrect data. The remaining correct sleep data is divided into sleep data with a disease tag and sleep data without a disease tag according to the data source, and these data are put into an artificial intelligence learning model for training.

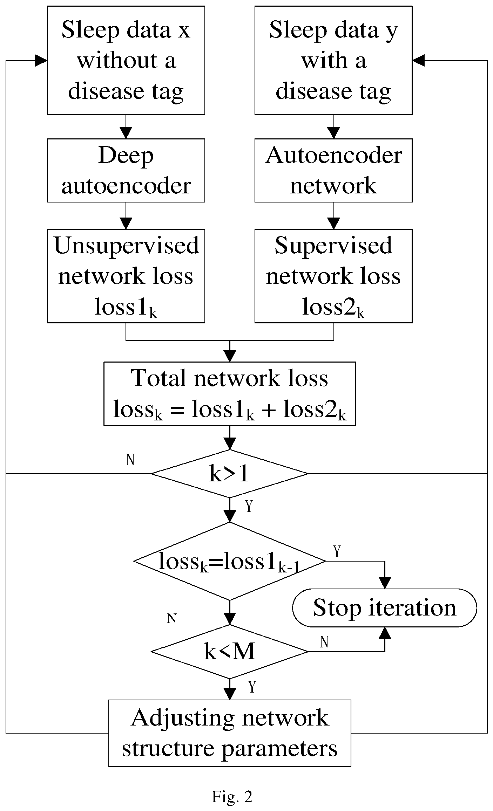

Fig. 2 shows a flow chart of data training performed by the artificial intelligence learning model in the embodiment of the present disclosure. As shown in Fig. 2, the process of inputting the correct data after data preprocessing in the artificial intelligence learning model for training comprises:
(1) After preprocessing, sleep data x without a disease tag is sent into a deep autoencoder of an autoencoder network, and an unsupervised network loss loss1ₖ is obtained by calculation. In the present application, an optimal calculation method is selected as follows: the sleep data x is sent into the deep autoencoder to obtain a mean square difference between inputs and outputs, and such a difference is assigned to the unsupervised network loss losslk.
(2) After preprocessing, sleep data y with a disease tag is put into an autoencoder network having the same parameters as the above autoencoder network to obtain intermediate layer dimension reduction output data, and a supervised network loss loss2k is obtained by calculation. In the present application, an optimal method for calculating the supervised network loss loss2ₖ is selected as follows: the intermediate layer dimension reduction output data as obtained is divided into different categories according to the disease tag. A central point of each category is found out, and distances from data of the same category to central points of the same category are calculated. A sum of the obtained data is assigned to the supervised network loss loss2ₖ.
(3) Loss1ₖ and loss2ₖ as obtained in above (1) and (2) are summed to obtain a total network loss lossₖ, i.e., lossₖ = loss1ₖ + loss2ₖ, and whole network structure parameters are adjusted using the back-propagation algorithm. Then, x and y are sent into the autoencoder network structure again by iterative training. Above step (1) and step (2) are repeated respectively to calculate an unsupervised network loss losslk+i and a supervised network loss loss2_{K+1} again after the adjustment of the network structure parameters, and a total network loss of this round lossₖ₊₁ = loss1ₖ₊₁ + loss2ₖ₊₁ is obtained by calculation.
(4) The losses after adjustment of the network structure parameters are compared and the network structure parameters are adjusted again until lossₖ₊₁ = lossₖ in the network structure or until a maximum number of iterations is reached. By the maximum number of iterations, it means that a maximum number of iterations M is set as required during training of the artificial intelligence learning model, and when the number of iterations is M (k=M), even if the total network loss still changes, iterative training will not continue.

So far, the training of the artificial intelligence learning model is completed, and a neural network loss and an autoencoder network loss obtained from the training will be used as physiological evaluation indexes to evaluate whether the individual corresponding to the sleep data is in a sick state or a normal health state.

The intermediate layer dimension reduction output data obtained when the sleep data y with a disease tag is sent into the autoencoder network will be further used as an input of an SVM classifier to train the classifier model, such that the classifier model can identify disease types corresponding to different data. The classifier model obtained by training can identify the specific type of disease of the individual generating the sleep data.

The physiological evaluation index and the classifier model obtained by the above training can be used to generate an individual health status analysis report corresponding to the sleep data generated. In specific implementation, the sleep data of an individual who needs a health assessment is selected from the cloud server, and put into the artificial intelligence learning model that has been trained. The disease status of the individual is determined by the model. If the result is that the individual is in a disease state, the disease type of the individual can be further obtained. Finally, a terminal device can be used to output or print out a human health assessment report containing the above assessment result, so as to achieve the purpose of using sleep data to predict diseases.

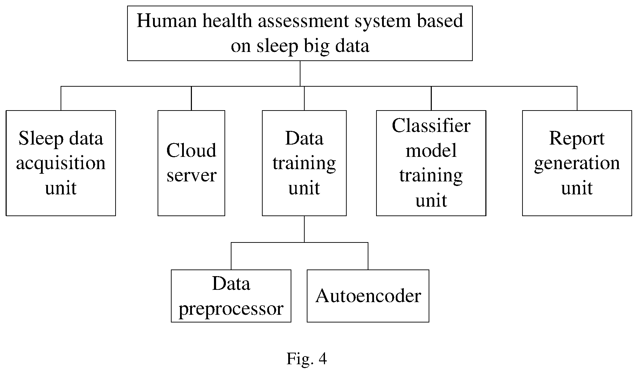

Fig. 4 is a frame diagram of a human health assessment system based on sleep big data according to an embodiment of the present disclosure. As shown in Fig. 4, the human health assessment system based on sleep big data comprises:
a sleep data acquisition unit for acquiring various physiological data of a human body during sleep, the data coming from a sensor mounted on a bed;
a sleep data storage unit, including a cloud server for storing all sleep data collected by the sleep data acquisition unit;
a data training unit for preprocessing sleep data and training data by an artificial intelligence learning model to obtain a physiological evaluation index, wherein the data training unit includes: a data preprocessor for filtering out missing and incorrect sleep data stored in the cloud server, and an autoencoder for creating an autoencoder network, and iteratively training the data by calculating network losses;
a classifier model training unit for performing classifier model training on the sleep data trained by the artificial intelligence learning model; and
a report generation unit for generating a human health status analysis report according to the human physiological evaluation index and the classifier model as obtained based on data training.

It should be noted that the system of the present disclosure can be arranged in a control system of an electric bed or mounted in a terminal device of a human-computer interaction operation. The device further comprises an operating mechanism for controlling the report generation unit to generate a report, such that a user can customize the assessment system as required. The terminal device described in the embodiments of the description can be an internet device such as a user device, a smart phone, a computer, a mobile internet device, or a wearable intelligent device, which is not limited in the embodiments of the present disclosure.

A person skilled in the art will appreciate that the embodiments of the present disclosure may be provided as a method, a system, or a computer program product. Therefore, the present disclosure may take the form of a complete hardware embodiment, a complete software embodiment, or an embodiment combining software and hardware aspects.

The above is only preferred embodiments of the present disclosure. It should be pointed out that a person of ordinary skill in the art can make some improvements and supplements without departing from the method of the present disclosure, and these improvements and supplements shall also be regarded as in the scope of protection of the present disclosure.

## Claims

1. A human health assessment method based on sleep big data, comprising the steps of:
acquiring various physiological data of a human body during sleep by a sensor mounted on a bed, and storing these sleep data permanently in a cloud server;
preprocessing the sleep data stored in the cloud server to filter out missing and incorrect data, and inputting correct data into an artificial intelligence learning model for training, such that the artificial intelligence learning model learns disease features and calculates to obtain a physiological evaluation index;
training a classifier model using the disease features of data learnt by the artificial intelligence learning model, such that the classifier model can identify disease types corresponding to different data; and
generating a human health status analysis report according to the human physiological evaluation index and the classifier model obtained in the data training.

2. The human health assessment method based on sleep big data according to claim 1, wherein the various physiological data of a human body during sleep include: heart rate, respiratory rate, turning over, fretting, snoring, and leaving bed information.

3. The human health assessment method based on sleep big data according to claim 1, wherein the correct data after preprocessing is divided into sleep data with a disease tag and sleep data without a disease tag, the sleep data with a disease tag coming from a human body suffering from a known disease type while the sleep data without a disease tag coming from a human body in an unknown disease status.

4. The human health assessment method based on sleep big data according to claim 3, wherein the process of inputting the correct data into the artificial intelligence learning model for training comprises:
sending the sleep data without a disease tag after preprocessing to an autoencoder network to calculate and obtain an unsupervised network loss;
sending the sleep data with a disease tag after preprocessing to an autoencoder network having same parameters as the autoencoder network, to obtain intermediate layer dimension reduction output data and calculate to obtain a supervised network loss; and
iteratively training above two steps, and stopping iteration until a sum of the unsupervised network loss and the supervised network loss as obtained no longer changes or reaches maximum iterations, sleep data obtained then being used as the physiological evaluation index.

5. The human health assessment method based on sleep big data according to claim 4, comprising: inputting the intermediate layer dimension reduction output data obtained from the sleep data with a disease tag in the autoencoder network into a classifier to train one classifier model.

6. The human health assessment method based on sleep big data according to claim 5, wherein the physiological evaluation index is used to evaluate whether the human body is in a sick state, and the classifier model is used to analyze a disease type of the human body.

7. A human health assessment system based on sleep big data, comprising:
a sleep data acquisition unit for acquiring various physiological data of a human body during sleep, the data coming from a sensor mounted on a bed;
a sleep data storage unit, including a cloud server for storing all sleep data collected by the sleep data acquisition unit;
a data training unit for preprocessing sleep data and training data by an artificial intelligence learning model to acquire a physiological evaluation index;
a classifier model training unit for performing classifier model training on the sleep data trained by the artificial intelligence learning model; and
a report generation unit for generating a human health status analysis report according to the human physiological evaluation index and the classifier model as obtained based on data training.

8. The human health assessment system based on sleep big data according to claim 7, wherein the data training unit comprises:
a data preprocessor for filtering out missing and incorrect sleep data stored in the cloud server; and
an autoencoder for creating an autoencoder network, and iteratively training data by calculating a network loss.

9. A terminal device, wherein the device comprises the human health assessment system based on sleep big data according to claim 7 or 8.

10. The terminal device according to claim 9, wherein the device further comprises an operating mechanism for controlling the report generation unit in claim 7 to generate a report.
